# EUROPÄISCHE PATENTSCHRIFT

(11) **EP 0 450 344 B1**
(45) Veröffentlichungstag und Bekanntmachung des Hinweises auf die Patenterteilung: **10.01.1996**
(21) Anmeldenummer: 91103626.7
(22) Anmeldetag: 09.03.1991
(51) Int. Cl.: A01H 4/00

(54) **Verfahren zur Herstellung von Pilocarpin aus in vitro-Kulturen von Pilocarpus**
Method for the production of pilocarpin from vitro-cultures of Pilocarpus
Procédé pour la production de pilocarpin par vitro-cultures de Pilocarpus

(30) Priorität: 23.03.1990 DE 4009392
(43) Veröffentlichungstag der Anmeldung: 09.10.1991
(73) Patentinhaber: MERCK PATENT GmbH, D-64271 Darmstadt (DE)
(72) Erfinder: Reuther, Gerhard, Prof. Dr., W-6222 Geisenheim (DE)

(56) Entgegenhaltungen:
- EP-A- 0 344 992
- GB-A- 2 231 585
- US-A- 3 761 595
- ACTA HORTICULTURAE Bd. 79, 1978, Seiten 113 - 126 A.J.ABBOT 'Practice and promise of micropropagation of woody species'
- J.M. BONGA 6 D.J. DURZAN -eds-: "Cell and tissue culture in forestry," 1987 Martinus Nijhoff Publ. DORDRECHT VOL.3, Case histories: Gymnosperms, Angiosperms and Palms; Chapter 17, pages 224-246, V. CHALUPA: "European hardwoods". *Seite 225, Zeile 24 - Seite 228, Zeile 10*.

## Beschreibung

Die Erfindung betrifft ein Verfahren zur Herstellung von Pilocarpin aus Suspensions- oder ausdifferenzierten in vitro-Pflanzenkulturen der Gattung Pilocarpus. Die vor allem in Südamerika beheimatete Gehölzpflanze der Gattung Pilocarpus mit ihren wichtigsten Vertretern P. jaborandi und P. microphyllus enthält unter anderem das Imidazol-Alkaloid Pilocarpin.

Pilocarpin wird als Pharmazeutikum bzw. Therapeutikum eingesetzt. Es ist ein direkt wirkendes Parasympathomimetikum. Es wirkt muscarin- und acetylcholinartig mit dem Vorteil, daß es nicht von Cholinesterase abgebaut werden kann. Es fördert die Sekretion der Schweiß- und Speicheldrüsen und regt die glatte Muskulatur des Darmes und der Bronchien an. Das fast alleinige Anwendungsgebiet ist heute die Ophthalmologie. Da es als Miotikum den intraokulären Druck herabsetzt, wird es vorzugsweise zur Behandlung des grünen Stars (Glaukom) eingesetzt.

Die chemische oder biochemische Gewinnung von Pilocarpin hat sich als schwierig und bislang unökonomisch herausgestellt. So wird der Bedarf an Pilocarpin weitgehend aus der Pflanze selbst durch Isolierung und Aufreinigung des Alkaloids gedeckt. Wie aber bei vielen anderen tropischen Pflanzen auch, bewahren die Samen von Pilocarpus ihre Keimfähigkeit nur sehr kurze Zeit. Innerhalb weniger Wochen ist die Keimrate um mehr als 90 % zurückgegangen. Dies bedeutet, daß die Kultivierung von Pilocarpus-Sämlingen nur in der Nähe der natürlichen Standorte erfolgreich durchgeführt werden kann. Ein weiterer Nachteil ist das langsame Wachstum von Sämlingen unter Gewächshausbedingungen.

Aus einer Publikation von A.J. Abbott (Acta Horticulturae, 79, 113-126 (1978)) ist bekannt, daß aus einem aus Organexplantaten gewonnenen Kallus in vitro mittels geeigneter, unterschiedlich zusammengesetzter Nährböden in bestimmten Fällen ausdifferenzierte Pflanzen kultviert werden können.

Andererseits schlugen Versuche bislang fehl, Pilocarpus in vitro zu kultivieren. Überhaupt ist der Erfolg einer in vitro-Kultivierung von Gehölzpflanzen, insbesondere aus Kallus, nur in Ausnahmefällen sichergestellt und dies auch nur bei relativ unproblematischen, anspruchslosen Arten (Bonga u. Durzan (1987), Cell and Tissue Culture in Forestry, Vol. 1-3, Martinus Nijhoff Publishers; Chalupa (1987) in: Bonga u. Durzan (1987), l.c.; Wann (1988), Horticultural Reviews 10, 153). Neben der schwierigen in vitro-Kultivierung als solche ist es überdies auch äußerst fragwürdig, ob die so erhaltenen in vitro-Kulturen noch in der Lage sind, das gewünschte Naturprodukt - in diesem Fall Pilocarpin - in akzeptablen Mengen zu synthetisieren. Zahlreiche solcher Kulturen haben sich als nicht praktikabel erwiesen.

Es bestand somit die Aufgabe, ein Verfahren zur Herstellung von Pilocarpin aus Pflanzenmaterial auf Basis einer zur Alkaloidbildung befähigten in vitro-Kultur zu entwickeln, so daß die oben genannten Nachteile bezüglich der Standort- und Wachstumsbedingungen vermieden werden können.

Es wurde nun gefunden, daß eine vermehrungsfähige und zur Bildung von Pilocarpin befähigte in vitro-Gewebekultur aus Explantaten von Organfragmenten von Samen und insbesondere jungen Keimlingen von Pilocarpus hergestellt werden kann. Es lassen sich sowohl Suspensions- als auch ausdifferenzierte Kulturen gewinnen. Dabei ist es als erfindungswesentlich anzusehen, daß bei der Herstellung der genannten Kulturen eine ganz bestimmte Zusammensetzung und Abfolge von Hormonen bzw. hormonhaltigen Nährmedien eingehalten werden muß.

Gegenstand der Erfindung ist somit ein Verfahren zur Herstellung von Pilocarpin durch Isolierung und Aufreinigung aus Pilocarpus, dadurch gekennzeichnet, daß man in vitro gezogene Kulturen einsetzt.

Gegenstand der Erfindung ist insbesondere ein entsprechendes Verfahren, das dadurch gekennzeichnet ist, daß man zur Gewinnung der in vitro-Kultur einen aus Organexplantaten stammenden Kallus auf einem geeigneten Nährboden herstellt, die Sproßdifferenzierung des Kallus auf unterschiedliche, hintereinander folgende, jeweils spezifische Hormone enthaltende Nährböden induziert und die so gewonnenen Zellen in einer Nährlösung oder nach Induzierung der Wurzelbildung als ausdifferenzierte Pflanze kultiviert.

Das erfindungsgemäße Verfahren wird vorzugsweise wie folgt durchgeführt.

Zur Kultivierung von Pilocarpus eignen sich vorzugsweise die Arten Pilocarpus jaborandi und Pilocarpus microphyllus, insbesondere aber P. microphyllus. Zunächst wird eine Kalluskultur in an sich bekannter Weise hergestellt. Man kann hierzu entweder Samen oder Organexplantate aus Keimlingen von Pilocarpus verwenden.

Die Kallusbildung aus Keimlingen ist allerdings bevorzugt, da die aus Samen hergestellten Kalli in der Regel nicht befähigt sind, ausreichend Sprossen zu bilden. Die Sproßvermehrung ist aber essentiell für das erfindungsgemäße Verfahren. Zur Gewinnung von Kallus aus Explantaten von Keimlingen werden vorzugsweise Keimlinge von 8 bis 12 cm Länge als Ausgangsmaterial eingesetzt. Als Primärexplantate eignen sich beispielsweise Sproßspitzenexplantate oder Blattstielsegmente. Aufgrund der größeren Regenerationsaktivität ist jedoch der Einsatz von Sproßspitzenexplantaten zur Kallusbildung im besonderen Maß bevorzugt. Die Explantate bzw. Keimlinge werden in an sich bekannter Weise präpariert bzw. sterilisiert und unter Belichtung auf ein festes Nährmedium bei Temperaturen zwischen 22 °C und 27 °C, vorzugsweise 25 °C, gegeben und nach Standardmethoden mehrere Wochen lang kultiviert. Diese Kultivierungsbedingungen sind auch für das gesamte erfindungsgemäße Verfahren maßgebend. Als Basis-Nährmedien können Medien verwendet werden, die für Gehölzpflanzen besonders geeignet sind, so beispielsweise das Woody Plant Medium (WPM) (LLoyd u. McCown (1980), Proc. Inter. Plant Prop. Soc. 30, 421) oder Varianten davon. Die qualitative und quantitative Zusammensetzung dieses Grundmediums (ohne Hormone) können in einem gewissen Bereich variiert werden, ohne daß das erfindungsgemäße Verfahren wesentlich beeinflußt wird. Insbesondere läßt sich die Menge an Zucker in einem größeren Bereich verändern (10-40 g/l, vorzugsweise 20 g/l). Das Basalmedium eignet sich sowohl für flüssige Medien als auch für feste Nährböden (Hinzugabe von Gelbildnern wie beispielsweise Agar oder Gelrite). Eine detaillierte Zusammensetzung ist in den Ausführungsbeispielen angegeben. Erfindungswesentlich ist unter anderem die qualitative und quantitative Zusammensetzung der Hormonbestandteile, die dem Basalmedium zugeführt werden müssen, damit eine optimale vegetative Vermehrung der Pilocarpusexplantate einsetzen kann.

Die Bildung von insbesondere organogenem Kallusgewebe aus den Pilocarpusexplantaten wird erfindungsgemäß durch die Zugabe der drei Hormone α-Naphthylessigsäure, N⁶-Dimethylallyladenin und Zeatin zu dem WPM-Basalmedium bewirkt. Die Konzentration beträgt für Naphthylessigsäure 0,75 bis 1,25 mg/l, vorzugsweise 1,0 mg/l, für Dimethyladenin 0,3 bis 0,7 mg/l, vorzugsweise 0,5 mg/l und für Zeatin ebenfalls 0,3 bis 0,7 mg/l, vorzugsweise 0,5 mg/l. Die Kultivierung auf dem hormonhaltigen Medium (M3-Medium) bewirkt eine Kallusbildung durchweg mit Organstrukturen verschiedenster Differenzierung. Überraschenderweise können durch die entsprechende Hormongabe unter anderem ausdifferenzierte somatische Embryonen induziert werden. Dies ist bislang bei Kallus vegetativer Pflanzenteile aus Gehölzen nicht, bzw. nicht in dem Ausmaß beobachtet worden. Auf dem M3-Medium werden aus dem organogenen Kallusgewebe vorzugsweise mehrere Subkulturen in an sich bekannter Weise hergestellt. Das Medium fördert im Zusammenwirken mit der Belichtung die Bildung von Organen in großer Zahl. Der Kallus besitzt auf diesem Medium eine hohe induzierte embryogene Potenz. Allerdings kann mit Hilfe des M3-Mediums keine ausreichende Entwicklung von Sprossen erzielt werden. Das Sproßwachstum ist jedoch für die weitere Entwicklung einer funktionsfähigen in vitro-Kultur von großer Bedeutung.

Die Sproßbildung kann nun überraschend durch Umsetzen des organogenen Kallusgewebes auf ein Nährmedium mit geänderter Hormonzusammensetzung bewirkt werden. Als Basalmedium ist beispielsweise wiederum das WPM-Medium in gleicher oder variierter Zusammensetzung, oder vergleichbare andere Medien geeignet. Anstelle der Hormonzusammensetzung des M3-Mediums enthält nach dem erfindungsgemäßen Verfahren das Folgemedium lediglich 6-Benzylaminopurin in einer Konzentration zwischen 0,3 und 0,8 mg/l, vorzugsweise aber zwischen 0,4 und 0,6 mg/l. Das Sproßwachstum teilweise bis zu Sproßbüscheln und die Sproßstreckung wird durch dieses hormonhaltige Medium (Betulamedium) induziert und/oder deutlich gefördert. Dabei trifft der Effekt in der Regel erst bei der zweiten und folgenden Subkultur verstärkt in Erscheinung. Generell beläuft sich die Dauer einer Subkultur auf 30 bis 60 Tage, vorzugsweise auf 40 Tage, je nach Pflanzenmaterial und äußeren Bedingungen, wie Temperatur, Beleuchtung usw. Der vorteilhafte Effekt der erfindungsgemäßen Hormonzugabe läßt sich insbesondere durch die Tatsache verdeutlichen, daß bei Rücksetzung der auf im zweiten Schritt Betulamedium angezogenen Kulturen, das Sproßwachstum sehr rasch auf ein Fünftel bis ein Zehntel reduziert wird.

Nach mehrere Subkulturen wird die so differenzierte Kultur gegebenenfalls auf ein neues, vorzugsweise WPM-Medium gesetzt, welches die Hormone 6-Benzylaminopurin und Indol-3-essigsäure enthält (DKW-Medium). 6-Benzylaminpurin wird dabei in einer Konzentration von 0,3 bis 0,8 mg/l, vorzugsweise von 0,4 bis 0,6 mg/l, und Indol-3-essigsäure in einer Konzentration von 0,05 bis 0,2 mg/l, vorzugsweise von 0,08 bis 0,15 mg/l eingesetzt. Das DKW-Medium verstärkt weiter die Sproßvermehrung und konditioniert überdies die Sprossen für eine spätere Bewurzelung. Eine solche Bewurzelung ist notwendig um in einem weiteren Verfahrensschritt eine ausdifferenzierte Pflanzenkultur zu erhalten. Zur Herstellung einer reinen Suspensionskultur, für die eine Bewurzelung der Sproßbündel unwesentlich ist, kann auch auf die Umsetzung der Kultur vom Betula- auf das DKW-Medium verzichtet werden, ohne daß dabei entscheidende Nachteile entstehen. Umgekehrt kann auch zur Herstellung einer ausdifferenzierten Pflanzenkultur notfalls auf das Betulamedium verzichtet werden. In jedem Fall ist für das erfindungsgemäße Verfahren wesentlich, daß die Medium-/Hormon-Reihenfolge M3/Betula oder M3/DKW, vorzugsweise aber in allen Fällen die Mediensequenz M3/Betula/DKW eingehalten wird. Der Einsatz des M3-Mediums als Primärmedium ist in jedem Fall essentiell, da eine Kultivierung der Explantate ausschließlich auf Betula oder DKW zur Folge hat, daß nicht ausreichende Kallusmasse gebildet bzw. diese aufgebraucht wird. Zur Herstellung von Suspensionskulturen werden die entsprechenden Betula- oder DKW-Medien ohne Gelbildner eingesetzt.

Die bekannten Methoden zur Bewurzelung von Sproßkulturen durch Anreicherung der Kulturmedien mit Auxinen führt bei Pilocarpus nicht zum Erfolg. Überraschenderweise kann die Bewurzelung aber durch folgende Verfahrensschritte induziert werden. Die Sprossen werden unter sterilen Bedingungen maximal 3 mm tief in eine wäßrige Auxinlösung getaucht. Es muß eine extrem hohe Auxinkonzentration von 0,8 bis 1,5 g/l, vorzugsweise 1,0 bis 1,2 g/l, eingesetzt werden. Als Auxine eignen sich beispielsweise Indolessigsäure, Indolbuttersäure oder α-Naphthylessigsäure. Besonders bevorzugt ist jedoch Indolbuttersäure. Die Eintauchdauer beträgt drei bis sechs Minuten, vorzugsweise 5 Minuten. Die so behandelte Sproßkultur wird vorzugsweise auf dem DKW-Medium vorkultiviert und anschließend auf ein hormonfreies Medium, beispielsweise WPM mit einem Gelbildner umgesetzt. Hierbei ist Gelrite gegenüber Agar bevorzugt, da es die Wurzelbildung der Pilocarpuskultur etwa um den Faktor 3 bis 5 erhöht. Ferner wird durch die Wurzelinduktion das Sproßwachstum gefördert. Die so erhaltenen ausdifferenzierten Pilocarpus in vitro-Kulturen lassen sich über zahlreiche Subkulturpassagen auf einem geeigneten Nährboden, vorzugsweise DKW, kultivieren. Sie können aber auch nach mehreren Subkulturpassagen in Erde umgesetzt werden und unter geeigneten Gewächshaus- bedingungen in an sich bekannter Weise aufgezogen werden.

Die Gewinnung von Pilocarpin kann erfindungsgemäß sowohl aus den Suspensionskulturen als auch aus den ausdifferenzierten Kulturen erfolgen. Die Isolierung und Aufreinigung des Alkaloids geschieht nach Standardmethoden und ist in den Ausführungsbeispielen näher dargelegt. Der Gehalt an aufgereinigtem Alkaloid beträgt bei den ausdifferenzierten Kulturen 0,01 bis 0,2 % (w/w) bezogen auf das Gesamtgewicht des eingesetzten Pflanzenmaterials, meist 0,05 bis 0,1 %. Das entspricht etwa einem Fünftel bis einem Zwanzigstel der Alkaloidmenge von natürlich aufgewachsenem Pilocarpus am Heimatstandort. Bei Suspensionskulturen liegt der Alkaloidgehalt noch etwa um den Faktor zehn tiefer als bei den ausdifferenzierten in vitro-Kulturen, also zwischen 0,001 % und 0,05 %, insbesondere zwischen 0,005 bis 0,02 % (w/w). Dies bedeutet, daß aus etwa 1 kg Suspensionskultur durchschnittlich 100 mg Pilocarpin isoliert werden können.

Pilocarpus microphyllus liefert nach dem erfindungsgemäßen Verfahren schneller und leichter geeignete in vitro-Kulturen mit entsprechend höheren Ausbeuten an Alkaloid als P. jaborandi.

### Beispiele:

### Beispiel 1:

### Herstellung einer Kalluskultur aus Samen

Samen von P. microphyllus werden 2 Stunden in einer wäßrigen Natriumhypochloritlösung (5 %) sterilisiert. Nachfolgend erfolgt 3maliges Waschen mit einem sterilen 1:1 Gemisch aus Leitungs- und entionisiertem Wasser 10, 20 und 30 Minuten lang. Die Samenschale wird abgeschält und die geschälten Samen werden längs halbiert und die Spalthälften werden mit der Schnittfläche auf ein M3-Medium (Zusammensetzung in Beispiel 2) gelegt und die Samen quer halbiert. Die Inkubation erfolgt im Dunkeln im Brutschrank bei 25-27 °C. Nach 7 Wochen werden gebildete Kallusstücke vom Primär- explantat (Wundränder) abgeschnitten und auf ein frisches M3-Medium gegeben. Die Kulturen werden bei etwa 25 °C im 16 Stundentag belichtet (3-4 K Lux Mischlicht aus Tageslicht- und Warmtonleuchter), herangezogen bis nach mehreren Subkulturpassagen (4-5 Wochen/Passage) eine grüne, sproßorganogene Kalluskultur vorliegt.

### Beispiel 2:

### Herstellung einer Kalluskultur aus Keimpflanzenexplantaten

Samen von Pilocarpus microphyllus werden in einem TKS/Sandgemisch unter Warmhausbedingungen zum Keimen gebracht. Nach Erreichen einer Höhe von ca. 10 cm werden die Keimlinge etwa 1 cm über der Erde abgeschnitten. Die Teile werden zur Dekontamination in 70 % Ethanol 20 Sekunden lang und anschließend 12 Minuten lang in 5%iger NaOCl-Lösung, der 1 Tropfen Tween 20 zugesetzt ist, getaucht. Es folgt 3maliges Waschen mit sterilem Wasser 2mal 10 Minuten und 1mal 30 Minuten lang. Von den Pflanzenteilen werden Sproßspitzen oder Sproßsegmente von 1 bis 3 mm Größe abgeschnitten und auf das unten angegebene M3-Medium gesetzt. Die Kulturen werden bei Licht im 16 Stundentag (3-4 K Lux Mischlicht aus Tageslicht- und Warmtonleuchter) bei 25 °C gehalten. Nach 10-12 Wochen bilden sich grünliche Kallusklumpen, die mehrfach in Subkultur genommen werden. Alle Arbeiten werden unter sterilen Bedingungen vorgenommen. Eine Subkulturpassage dauert 4-5 Wochen. Die Kallusmenge nimmt ständig zu. Der Kallus besitzt teilweise ausgeprägte Organanlagen, unter anderem auch ausdifferenzierte somatische Embryonen. Sproßwachstum ist jedoch kaum vorhanden.

| WPM-Medium (hormonfrei): | | | |
|---|---|---|---|
| Makroelemente: | mg/l | Mikroelemente: | mg/l |
| K₂SO₄ | 990 | H₃BO₃ | 6,2 |
| NH₄NO₃ | 400 | MnSO₄ . H₂O | 16,9 |
| CaCl₂ . 2 H₂O | 96 | oder | |
| Ca(NO₃)₂ . 4 H₂O | 556 | MnSO₄ . 4 H₂O | 22,3 |
| MgSO₄ . 7 H₂O | 370 | NaMoO₄ . 2 H₂O | 0,25 |
| KH₂PO₄ | 170 | CuSO₄ . 5 H₂O | 0,25 |
| FeSO₄ . 7 H₂O | 27,8 | | |
| Na₂EDTA | 37,3 | | |

| Organische Bestandteile: | |
|---|---|
| Glycin | 2,0 |
| Nicotinsäure | 0,5 |
| Pyridoxin-HCl | 0,5 |
| Thiamin-HCl | 1,0 |
| Meso-Inosit | 100 |
| Saccharose | 20 g/l |
| Difco-Agar | 7 g/l (entfällt bei Suspensionskulturen) |

| M3-Medium: | |
|---|---|
| WPM-Medium (hormonfrei) | |
| α-Naphthylessigsäure | 1,0 mg/l |
| N⁶-Dimethylallyladenin | 0,5 mg/l |
| Zeatin | 0,5 mg/l |

### Beispiel 3:

### Herstellung einer Suspensionskultur

a) Die gemäß Beispiel 1 oder 2 hergestellte organogene Kalluskultur wird unter sterilen Bedingungen auf ein flüssiges Betulamedium (ohne Agar oder Gelrite) gesetzt und in mehreren Subkulturpassagen (ca. 2-4 Wochen/Passage) unter den in Beispiel 1 und 2 angegebenen Bedingungen sowie unter ständigem leichten Schütteln kultiviert. Es setzt eine intensive Sproßentwicklung und -vermehrung ein.
Betula-Medium:
WPM-Medium (hormonfrei, analog Beispiel 2, ohne Agar)

| | |
|---|---|
| 6-Benzylaminopurin | 0,5 mg/l |

b) Die gemäß Beispiel 3a erhaltene Suspensionskultur wird auf ein flüssiges DKW-Medium gesetzt und in mehrfachen Subkulturpassagen kultiviert.
DKW-Medium:
WPM-Medium (hormonfrei, analog Beispiel 2, ohne Agar + zusätzlich 10 g/l Saccharose)

| | |
|---|---|
| 6-Benzylaminopurin | 0,5 mg/l |
| Indol-3-essigsäure | 0,1 mg/l |

### Beispiel 4:

### Herstellung einer bewurzelten Pflanzenkultur

a) Die gemäß Beispiel 1 oder 2 hergestellte Kalluskultur wird unter sterilen Bedingungen auf ein Betulamedium (analog Beispiel 3a + Agar) gegeben und in mehreren Subkulturpassagen (ca. 4-5 Wochen/Passage) unter den in Beispiel 1 und 2 angegebenen Bedingungen kultiviert. Es setzt eine intensive, zu Büscheln neigende Sproßentwicklung und -vermehrung ein.
b) Die gemäß a) hergestellten Sproßkulturen werden auf ein DKW-Medium (analog Beispiel 3b + Agar) gesetzt und mehrfach wie unter a) subkultiviert. Die Kulturen sind jetzt für die Bewurzelung konditioniert.
c) Die gemäß Beispiel 3b oder 4b hergestellten Sproßkulturen werden wie folgt behandelt:
   - 5 Minuten langes Eintauchen der äußersten Spitze (ca. 2-3 mm) der Sprossen in eine wäßrige Lösung von Indolbuttersäure (1000 mg/l),
   - Anziehen einer Sproßvorkultur analog Beispiel 4b,
   - Umsetzen auf hormonfreies WPM-Medium mit Gelrite 3 g/l bei einer Sproßgröße von 15 bis 20 mm,
   - gegebenenfalls Umsetzen in Erde und allmähliche Gewöhnung an nicht sterile Bedingungen.

### Beispiel 5:

### Isolierung und Aufreinigung von Pilocarpin

320 g des gemäß Beispiel 4 gewonnenen Pflanzenmaterials wird bei 30 °C getrocknet und anschließend fein gemahlen. Man nimmt das Material in 400 ml Chloroform und 25 ml 10%iger Ammoniaklösung auf und rührt 20 Minuten lang. Danach filtriert man durch einen Wattebausch in einen Scheidetrichter, der 250 ml 5%ige Schwefelsäure enthält, und schüttelt ca. 1 Minute lang. Nach Phasentrennung wird die Chloroformphase verworfen. Der wäßrige drogenhaltige Rückstand wird nochmals mit 250 ml frischem Chloroform extrahiert, über einen Wattebausch filtriert und mit 250 ml 5%iger Schwefelsäure ausgeschüttelt. Die wäßrige Lösung wird am Rotationsverdampfer vorsichtig auf etwa die Hälfte des Volumens eingeengt. In der Kälte beginnt Pilocarpinhydrochlorid auszukristallisieren. Ausbeute 285 mg.

Der analytische Nachweis erfolgt über HPLC:

| | |
|---|---|
| Säule: | LiChrocart 125-4-RP-Select B®, mit LiChrospher 60®, 5 µm (Fa. E. Merck, Darmstadt, FRG) |
| Eluens: | 5%ige wäßrige KH₂PO₄-Lösung, pH 2,5 |
| Säulentemperatur: | 45 °C |
| Fließgeschwindigkeit: | 2 ml/Minute |
| Detektion: | 216 nm |

## Patentansprüche

1. Verfahren zur Herstellung von Pilocarpin durch Isolierung und Aufreinigung aus einer *in vitro* gezogenen Suspensions- oder ausdifferenzierten Kultur von *Pilocarpus*, wobei man zur Gewinnung der *in vitro* Kultur einen aus Organexplantaten stammenden Kallus auf einem geeigneten Nährboden herstellt, die Sproßdifferenzierung des Kallus auf unterschiedlichen, hintereinander folgenden, jeweils spezifische Hormone enthaltende Nährböden induziert und die so gewonnenen Zellen in einer Nährlösung oder nach Induzierung der Wurzelbildung als ausdifferenzierte Pflanze kultiviert, dadurch gekennzeichnet, daß man den aufeinander folgenden Nährböden/-medien die Hormone
- α -Naphtylessigsäure, N⁶-Dimethylallyladenin und Zeatin,
- 6-Benzylaminopurin und / oder
- 6-Benzylaminopurin und Indol-3-essigsäure
nacheinander in der angegebenen Reihenfolge zusetzt.

2. Verfahren nach Anspruch 1, dadurch gekennzeichnet, daß man den aufeinander folgenden Nährböden/ -medien die Hormone
- α -Naphtylessigsäure, N⁶-Dimethylallyladenin und Zeatin und
- 6-Benzylaminopurin
nacheinander in der angegebenen Reihenfolge zusetzt.

3. Verfahren nach Anspruch 1 oder 2, dadurch gekennzeichnet, daß die Wurzelbildung durch Behandlung mit Auxinen einer Konzentration von 0.8 bis 1.5 g/l induziert wird.

4. Verfahren nach Anspruch 3, dadurch gekennzeichnet, daß als Auxin Indolbuttersäure eingesetzt wird.

5. Verfahren nach einem der Ansprüche 1 bis 4, dadurch gekennzeichnet, daß man *Pilocarpus microphyllus* einsetzt.

## Claims

1. Process for the preparation of pilocarpine by isolation and purification of an *in-vitro* suspension culture or differentiated culture of *Pilocarpus*, where, to obtain the *in vitro* culture, a callus which is derived from organ explants is produced on a suitable nutrient medium, shoot differentiation of the callus is induced on a series of various nutrient media, each of which contains specific hormones, and the cells thus obtained are cultured in a liquid nutrient solution or, after root formation has been induced, as a differentiated plant, characterised in that the hormones
- α-naphthylacetic acid, N⁶-dimethylallyladenine and zeatine,
- 6-benzylaminopurine and/or
- 6-benzylaminopurine and indole-3-acetic acid
are added in succession in the sequence indicated to the nutrient media used in succession.

2. Process according to Claim 1, characterised in that the hormones
- α-naphthylacetic acid, N⁶-dimethylallyladenine and zeatine and
- 6-benzylaminopurine
are added in succession in the sequence indicated to the nutrient media used in succession.

3. Process according to Claim 1 or 2, characterised in that root formation is induced by a treatment with auxins in a concentration of 0.8 to 1.5 g/l.

4. Process according to Claim 3, characterised in that indole butyric acid is employed as the auxin.

5. Process according to one of Claims 1 to 4, characterised in that *Pilocarpus microphyllus* is employed.

## Revendications

1. Procédé pour préparer de la pilocarpine par isolement et purification à partir d'une culture, en suspension ou différenciée, effectuée in vitro, de Pilocarpus, où, pour obtenir la culture in vitro, on prépare sur un bouillon nutritif approprié un callus provenant d'explants d'organe, on provoque la différenciation des tiges du callus sur différents bouillons nutritifs, successifs, dont chacun contient des hormones spécifiques, et on cultive les cellules ainsi obtenues, dans une solution nutritive ou après induction de la rhizogenèse, sous forme d'une plante différenciée, caractérisé en ce qu'on ajoute successivement, dans l'ordre indiqué, aux bouillons/milieux nutritifs successifs, les hormones suivantes :
- acide α-naphtylacétique, N⁶-diméthylallyladénine et zéatine,
- 6-benzylaminopurine et/ou
- 6-benzylaminopurine et acide indole-3-acétique

2. Procédé selon la revendication 1, caractérisé en ce qu'on ajoute successivement dans l'ordre indiqué aux bouillons/milieux nutritifs successifs les hormones suivantes :
- acide α-naphtylacétique, N⁶-diméthylallyladénine et zéatine, et
- 6-benzylaminopurine.

3. Procédé selon la revendication 1 ou 2, caractérisé en ce que la rhizogenèse est induite par traitement avec des auxines ayant une concentration de 0,8 à 1,5 g/l.

4. Procédé selon la revendication 3, caractérisé en ce qu'on utilise comme auxine l'acide indole-butyrique.

5. Procédé selon l'une des revendications 1 à 4, caractérisé en ce qu'on utilise Pilocarpus microphyllus.
